# FASCICULE DE BREVET EUROPEEN

(11) **EP 0 558 598 B1**
(45) Date de publication et mention de la délivrance du brevet: **05.10.1994**
(21) Numéro de dépôt: 92900242.6
(22) Date de dépôt: 14.11.1991
(51) Int. Cl.: A61F 2/16

(54) **IMPLANT CRISTALLINIEN DE CHAMBRE POSTERIEURE**
IMPLANTIERBARE HINTERKAMMERLINSE
POSTERIOR CHAMBER LENS IMPLANT

(30) Priorité: 14.11.1990 FR 9014477
(43) Date de publication de la demande: 08.09.1993
(73) Titulaire: FRANCESCHI, François, F-13006 Marseille (FR)
(72) Inventeur: FRANCESCHI, François, F-13006 Marseille (FR)
(74) Mandataire: Marek, Pierre, Cabinet Marek
(86) Numéro de dépôt international: FR9100895
(87) Numéro de publication internationale: WO9208422

(56) Documents cités:
- WO-A-87/05797
- US-A- 4 244 060
- US-A- 4 661 108
- US-A- 4 738 680
- US-A- 4 764 169
- US-A- 5 002 568

## Description

La présente invention concerne un implant cristallinien ou lentille intraoculaire de chambre postérieure à fixation capsulaire, destiné à se substituer au cristallin naturel cataracté, et à corriger l'hypermétropie engendrée par l'aphakie.

L'extraction extracapsulaire du cristallin et son remplacement par un cristallin artificiel constitué par un implant cristallinien de chambre postérieure disposé à la place même du cristallin originel, constituent actuellement la technique la plus évoluée et la plus performante de la chirurgie de la cataracte.

Aussi minutieux qu'il soit, au cours de l'intervention d'extraction extracapsulaire du cristallin, le nettoyage du sac capsulaire du cristallin, après l'extraction du cristallin, ne permet pas d'éliminer la totalité des cellules germinatives cristalliniennes. Celles-ci prolifèrent plus ou moins rapidement postérieurement à l'intervention en provoquant une opacification progressive de la capsule postérieure désignée sous le nom de cataracte "secondaire" et qui nécessite une discission de cette capsule opaque au laser YAG ou à l'aiguille.

On a déjà proposé (US-A-4.661.108), dans le but de diminuer la migration de l'épithélium ou les fuites des fibres vitreuses, une lentille intraoculaire dont l'optique comporte, sur sa face postérieure, au moins deux saillies circulaires concentriques s'étendant vers l'arrière à partir de ladite face postérieure et séparées par une rainure circulaire, ces saillies de hauteur égale ayant pour objet de maintenir un espace libre entre la face postérieure de l'optique et la capsule postérieure, tout en formant un double joint d'étanchéité ou double barrière lorsque la lentille se trouve placée au contact de ladite capsule postérieure, de façon à contrarier la migration des cellules endothéliales ou de l'épithélium cortical.

Selon ce dispositif, les sommets plats des saillies circulaires sont disposés dans le même plan, ce qui impose un aplanissement complet de la capsule postérieure lequel ne respecte pas l'anatomie capsulaire, cela pouvant être une source de mal position ou de fibrose capsulaire et ne permettant pas de remplir efficacement le rôle d'une double barrière s'opposant à la migration des cellules germinatives cristalliniennes. D'autre part, la gorge ménagée entre les saillies circulaires n'a d'autre fonction que la séparation de ces dernières, de sorte qu'elle ne joue aucun rôle dans l'efficacité du double joint d'étanchéité recherché par la prévision d'une double barrière.

En outre, les dispositions préconisées dans le document US-A-4.661.108 ne sont pas applicables aux implants modernes comportant une optique bi-convexe.

La présente invention a pour objet de remédier aux insuffisances et inconvénients des implants cristalliniens de chambre postérieure connus et, notamment, aux implants du genre de celui qui est décrit dans le document susmentionné.

Cet objectif est atteint grâce à un implant cristallinien ou lentille intraoculaire comprenant, de manière classique, une optique discoïdale, généralement ronde, et deux anses ouvertes d'appui ou haptique, cette optique comportant, sur sa face postérieure, une gorge annulaire délimitée par deux anneaux saillants concentriques disposés au voisinage de la périphérie de ladite optique, cet implant cristallinien étant notamment remarquable par le fait que les sommets des anneaux saillants concentriques délimitant ladite gorge sont situés dans des plans différents, l'anneau saillant intérieur ayant une hauteur supérieure à la hauteur de l'anneau saillant extérieur.

En cours d'intervention de mise en place de cet implant cristallinien, la capsule postérieure du cristallin détendue pénètre, en partie, dans la gorge ou dépression circulaire, et vient épouser plus ou moins, la forme de cette dernière. Par la suite, avec le temps, la capsule postérieure se synéchiant progressivement au volet capsulaire antérieur est retendue. Cela provoque un effet de dépression dans le volume clos constitué par la gorge coiffée par la capsule postérieure. Cette dépression se traduit par un accolement étroit entre la capsule et les bords de la gorge constitués par les sommets des deux anneaux saillants. Cet accolement étroit permet de ralentir la progression par multiplication des cellules germinatives cristalliniennes, qui se fait classiquement de l'équateur du sac capsulaire, vers le centre ou axe optique, et qui est à l'origine d'une opacification secondaire de la capsule postérieure ou cataracte secondaire. L'implant cristallinien selon l'invention permet donc de retarder l'apparition de l'état de cataracte secondaire. En outre, cet implant cristallinien respecte l'anatomie capsulaire, n'entraine aucune mal position et ne provoque aucune fibrose capsulaire.

L'invention est décrite plus en détail dans l'exposé qui suit, en se référant aux dessins annexés dans lesquels :
La figure 1 est une vue de face d'un exemple d'exécution de l'implant cristallinien réalisé selon l'invention et considéré selon sa face postérieure.
La figure 2 est une vue en coupe diamétrale selon la ligne 2-2 de la figure 1.
La figure 3 est une vue de détail, à plus grande échelle et en coupe diamétrale, montrant le profil de la gorge annulaire de l'implant cristallinien.
La figure 4 est une vue comparable à la figure 2 et montrant un implant cristallinien comportant une lentille plan convexe.
La figure 5 est une vue analogue aux figures 2 et 4 et illustrant un implant cristallinien comportant une lentille concave convexe.
Les figures 6 et 7 illustrent le fonctionnement de la ventouse circulaire ménagée sur la face postérieure de l'optique.

On se réfère auxdits dessins pour décrire des exemples d'exécution intéressants, mais non limitatifs, de la lentille intraoculaire de chambre postérieure à fixation capsulaire ou implant cristallinien selon l'invention.

Cet implant cristallinien est constitué, de manière connue, par une pièce de matière plastique transparente composée d'une partie discoïdale centrale 1 dénommée l'optique et possédant une puissance optique, et d'une partie périphérique appelée l'haptique et le plus souvent formée par deux anses ouvertes 2, souples et élastiques, destinées à l'appui de l'implant sur les structures oculaires. Il peut être avantageusement réalisé en polyméthylméthacrylate, matériau transparent, bien toléré par l'organisme et filtrant les rayons ultraviolets.

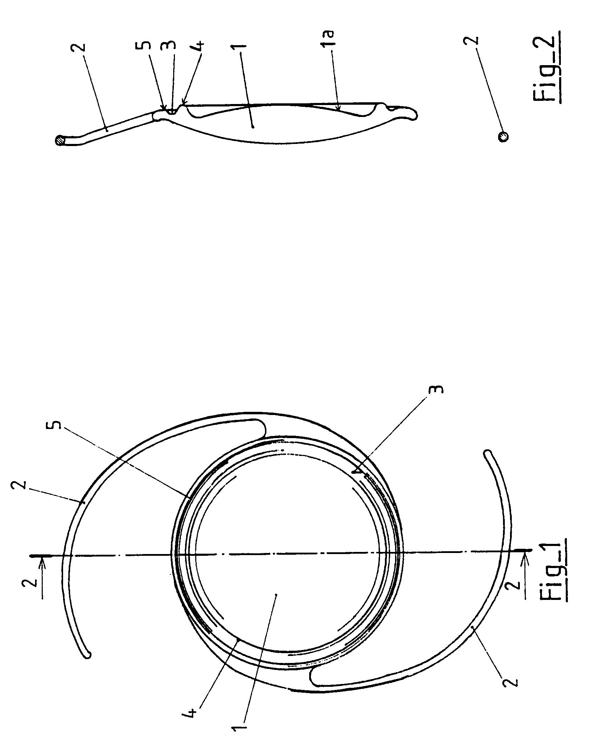

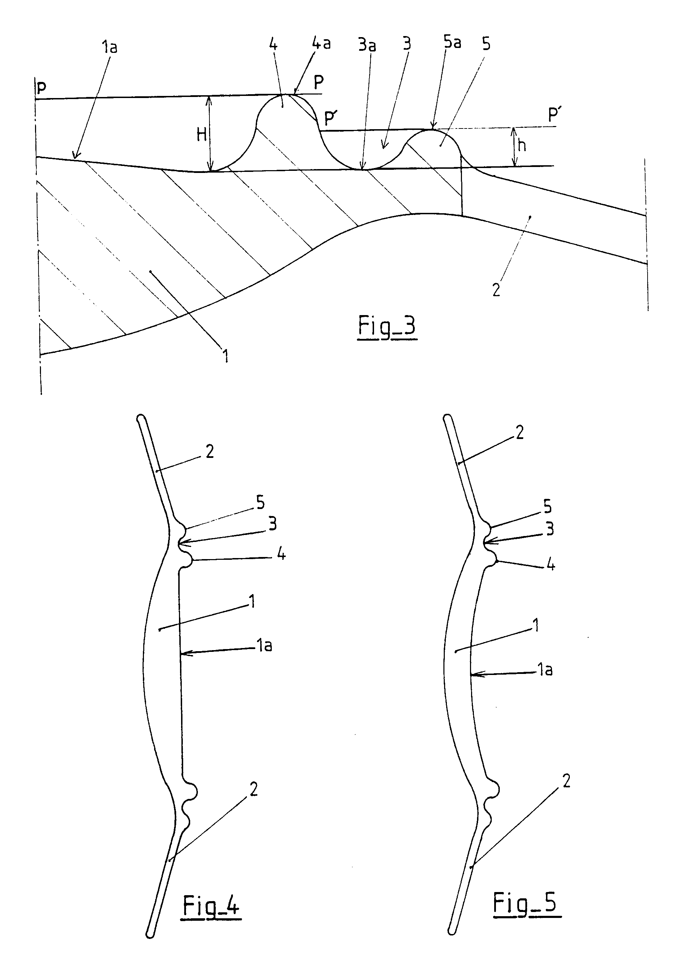

L'optique ronde 1 peut être réalisée sous forme d'une lentille optique pouvant avoir différentes formes telle que biconvexe (figure 2), plan convexe (figure 4), concave convexe (figure 5), etc., suivant la qualité de la vision de l'oeil destiné à recevoir l'implant.

L'optique 1 comporte, sur sa face postérieure destinée à être placée en contact avec la capsule postérieure du sac cristallinien, une gorge ou dépression annulaire 3, par exemple circulaire.

Cette gorge 3 est avantageusement prévue à proximité de la périphérie de l'optique 1.

L'implant cristallinien selon l'invention est remarquable par le fait que la gorge circulaire 3 est dimensionnée et conformée de manière à permettre, lors de sa mise en place dans le sac capsulaire, une pénétration, à l'intérieur de celle-ci, d'une portion circulaire de la capsule postérieure détendue, et à constituer ensuite une ventouse circulaire, comme cela est expliqué dans la suite du présent exposé.

De manière avantageuse, l'optique 1 comporte, à proximité de sa périphérie, deux bourrelets ou anneaux saillants concentriques et espacés 4 et 5 orientés vers l'arrière et s'étendant à partir de la face postérieure de ladite optique. Ces anneaux saillants 4, 5 dont les sommets, respectivement 4a et 5a, présentent un profil courbe ou arrondi, délimitent la gorge 3 dont le fond 3a présente également, de préférence, un profil courbe ou arrondi.

Selon une importante disposition caractéristique de l'invention, les sommets 4a, 5a, des anneaux saillants 4, 5 sont disposés dans des plans P-P, P'-P', différents. Plus précisément, l'anneau saillant intérieur 4 présente une hauteur H supérieure à la hauteur h de l'anneau saillant extérieur 5, par rapport au fond 3a de la gorge 3.

L'anneau saillant intérieur 4 a, par exemple, une hauteur de l'ordre de 0,40 mm, tandis que l'anneau saillant extérieur 5 a, par exemple, une hauteur de 0,20 mm, par rapport au fond 3a de la gorge 3. En outre, la distance séparant les anneaux saillants 4 et 5 à leur apogée est, par exemple, de l'ordre de 0,80 mm.

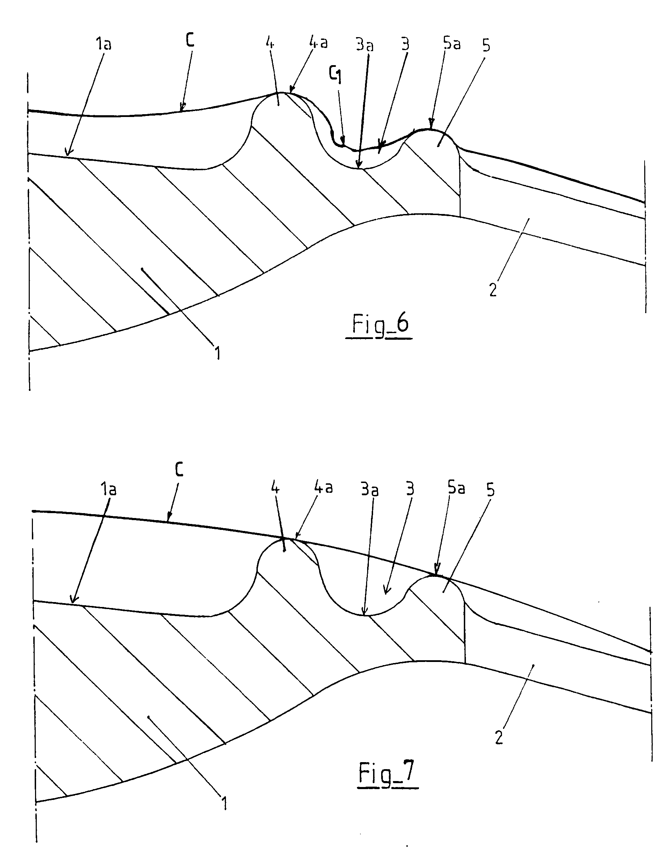

Les figures 6 et 7 illustrent le mode d'action de la ventouse postérieure dont est pourvue l'optique de l'implant, sur sa face arrière.

Lors de la mise en place de l'implant cristallinien dans le sac capsulaire, une portion circulaire ou sensiblement circulaire C1 de la capsule postérieure C détendue ou relâchée, pénètre à l'intérieur de la gorge 3 dont elle épouse plus ou moins la forme (figure 6).

Par la suite, avec le temps, la capsule postérieure C se synéchie progressivement au volet capsulaire antérieur et se trouve retendue. L'augmentation du volume clos constitué par la gorge 3 coiffée par la capsule postérieure C résultant de la tension de cette dernière, provoque un effet de vide ou de dépression dans ce volume clos. Cette dépression se traduit par une aspiration permettant de réaliser un accolement étroit entre la capsule postérieure C et les sommets 4a, 5a des deux anneaux saillants 4, 5, respectivement (figure 7), cet accolement étroit produisant le résultat recherché précédemment exposé ; l'efficacité et la permanence de ce résultat très avantageux étant grandement améliorées par la disposition et par la conformation des anneaux saillants délimitant la ventouse circulaire.

## Revendications

1. Implant cristallinien de chambre postérieure, comprenant une optique discoïdale (1) et une haptique (2), cette optique discoïdale (1) comportant, sur sa face postérieure et au voisinage de sa périphérie, une gorge annulaire (3) délimitée par deux anneaux saillants concentriques (4, 5) et dimensionnée de manière à autoriser, lors de sa mise en place dans le sac capsulaire, une pénétration, à l'intérieur de celle-ci, d'une portion circulaire (C1) de la capsule postérieure détendue (C), caractérisé en ce que les sommets (4a, 5a) des anneaux saillants concentriques (4, 5) délimitant ladite gorge (3) sont situés dans des plans (P-P, P'P') différents, l'anneau saillant intérieur (4) ayant une hauteur (H) supérieure à la hauteur (h) de l'anneau saillant extérieur (5), cette gorge ainsi dimensionnée et conformée constituant, après sa mise en place,une ventouse circulaire produisant un effet d'aspiration sur ladite capsule postérieure lorsque cette dernière se retend sous l'action de sa synéchie progressive au volet capsulaire antérieur.

2. Implant cristallinien suivant la revendication 1, caractérisé en ce que les sommets (4a, 5a) des anneaux saillants (4, 5) présentent un profil arrondi.

3. Implant cristallinien suivant l'une des revendications 1 ou 2, caractérisé en ce que le fond (3a) de la gorge ou dépression circulaire (3) présente un profil arrondi.

## Claims

1. Crystalline lens implant for the posterior chamber, comprising a discoid optical element (1) and a haptic element (2), said discoid optical element (1) having, on its posterior surface and in proximity to its edge, an annular recess (3) delimited by two concentric salient rings (4, 5) of dimensions such that they enable a circular portion (C1) of the relaxed posterior capsule (C) to penetrate inside it when placed in position in the capsular sac,
characterised in that the crowns (4a, 5a) of the concentric salient rings (4, 5) delimiting said recess (3) are situated in different planes (P-P, P'P'), the inner salient ring (4) having a height (H) greater than the height (h) of the outer salient ring (5), said recess thus dimensioned and configured constituting, once it has been placed in position, a circular vent exerting an aspirating effect on said posterior capsule whenever the latter retightens itself under the action of its progressive synechia to the anterior capsular flap.

2. Crystalline lens implant according to claim 1, characterised in that the crowns (4a, 5a) of the salient rings (4, 5) present a rounded profile.

3. Crystalline lens implant according to either of claims 1 and 2, characterised in that the bottom (3a) of the circular recess or depression (3) presents a rounded profile.

## Patentansprüche

1. Kristallines Implantat für die hintere Kammer, umfassend eine diskusförmige Optik (1) und eine Haptik (2), wobei diese diskusförmige Optik (1) auf ihrer Rückseite und ihrem Rand benachbart eine kreisförmige Kehle (3) aufweist, die durch zwei vorspringende konzentrische Ringe (4, 5) begrenzt wird und so dimensioniert ist, daß sie während des Einsetzens in den Kapselsack ein Eindringen eines kreisförmigen Teiles (C1) der spannungslosen hinteren Kapsel (C) in ihr Inneres erlaubt, dadurch gekennzeichnet, daß die Scheitelpunkte (4a,5a) der die Kehle (3) begrenzenden vorspringenden konzentrischen Ringe (4, 5) in unterschiedlichen Ebenen (P-P, P'P') angeordnet sind, wobei der vorspringende innere Ring (4) eine Höhe (H) aufweist, die größer ist als die Höhe (h) des vorspringenden äußeren Ringes (5), wobei diese so dimensionierte und gebildete Kehle nach ihrem Einsetzen einen kreisförmigen Saugnapf bildet, der einen Ansaugeffekt auf die hintere Kapsel erzeugt, während sich diese unter Einwirkung ihres zunehmenden Verwachsens an dem vorderen Kapselblatt nachspannt.

2. Kristallines Implantat nach Anspruch 1, dadurch gekennzeichnet, daß die Scheitelpunkte (4a, 5a) der vorspringenden Ringe (4, 5) ein rundes Profil aufweisen.

3. Kristallines Implantat nach einem der Ansprüche 1 oder 2, dadurch gekennzeichnet, daß der Boden (3a) der Kehle oder der kreisförmigen Vertiefung (3) ein rundes Profil aufweist.
